Europäisches Patentamt

European Patent Office

Office européen des brevets

(11) Publication number: **0 602 285 A1**

# EUROPEAN PATENT APPLICATION

(21) Application number: **92203911.0**

(22) Date of filing: **14.12.92**

(51) Int. Cl.5: **G01N 33/08**, A01K 43/00, G01N 29/04

(43) Date of publication of application:
**22.06.94 Bulletin 94/25**

(84) Designated Contracting States:
**AT BE CH DE DK ES FR GB GR IE IT LI LU MC NL PT SE**

(71) Applicant: **FPS Food Processing Systems B.V.
Stationsweg 117
NL-3771 VE Barneveld(NL)**

(72) Inventor: **Schouenborg, Kurt Otto Peter
Bakkehegnet 7
DK-3500 Vaerloese(DK)**

(74) Representative: **Smulders, Theodorus A.H.J.,
Ir. et al
Vereenigde Octrooibureaux
Nieuwe Parklaan 97
NL-2587 BN 's-Gravenhage (NL)**

(54) Method for detecting hair cracks or holes in egg shells, and apparatus for carrying out this method.

(57) Method and apparatus for detecting hair cracks or holes in egg shells, in which the egg shell to be tested is caused to vibrate locally by means of at least one striking element equipped with a vibration-sensitive element and a signal transducer, the vibration being transmitted via the vibration-sensitive element to the signal transducer and converted to an electrical signal whose amplitude and/or frequency and/or duration are a measure for the resilience of the egg shell. The signal generated when the striking element first strikes the egg is used for determining the egg diameter-dependent, variable distance between a fixed starting position of the striking element and the egg shell, whereafter the striking element is retracted over a certain fixed distance from the contact position to a reference position, from which position the striking element is contacted again with the egg shell for generating a measuring signal.

FIG.1

EP 0 602 285 A1

This invention relates to a method for detecting hair cracks or holes in egg shells, in which the egg shell to be tested is caused to vibrate locally by means of at least one striking element equipped with a vibration-sensitive element and a signal transducer, the vibration being transmitted via the vibration-sensitive element to the signal transducer and converted to an electrical signal whose amplitude and/or frequency and/or duration are a measure for the resilience of the egg shell.

Such a method is known from European patent application 0.295.755. In this known method, the striking element is moved from a fixed starting position in the direction of the egg. In the case of smaller eggs, therefore, before the striking element contacts the egg shell, a longer path must be traversed than in the case of larger eggs. The striking element can be subject to greater or lesser accelerations, depending on the distance to be traversed, and accordingly strike against the egg shell at a greater or lesser impulse of impact, which influences the shape of the measuring signal generated by the striking element. Since this measuring signal is compared with a standard measuring signal for an intact egg, differences between measuring signal and standard signal will occur that are not caused by the egg shell resilience to be measured, so that the accuracy of the measurement is adversely affected.

A second drawback of this known method is that the striking element must be returned to the starting position after each measurement. Since the eggs are not measured at a single point but at a large number of points located on a circumferential circle of an egg - for which purpose the eggs are rotatably supported - the striking element must be returned to the starting position as often as measurements are to be carried out. According as the distance between the egg shell and the starting position of the striking element is greater, the test cycle will be prolonged, considerably so in the case of small eggs, which adversely affects the processing capacity of the apparatus for carrying out the method.

The object of the invention is to provide a method which can supply a measuring signal that is independent of the distance between the striking element in its starting position and the egg shell to be tested and whereby the test cycle can be shortened. This object is accomplished according to the invention in that the signal generated when the striking element first strikes the egg is used for determining the egg diameter-dependent, variable distance between a fixed starting position of the striking element and the egg shell, whereafter the striking element is retracted over a certain fixed distance from the contact position to a reference position from which the striking element is contacted again with the egg shell for generating a measuring signal.

In this way a fixed distance has been created between the egg shell and the tip of the striking element, as a result of which the measuring signal to be generated by the striking element has become independent of the distance to be covered during the striking action because this distance is constant. Since the reference position of the striking element is located closer to the egg shell to be tested than is the starting position of the striking element, the test cycle is reduced considerably.

The apparatus known from European patent application 0.295.755 for detecting hair cracks or holes in egg shells comprises a carrying frame in which a series of striking elements are mounted so as to be axially movable, which striking elements have their centrelines directed to an egg shell to be tested and which, at the end proximal to the egg shell, are provided with a vibration-sensitive element, each striking element further comprising a signal transducer for converting the vibration transmitted by the vibration-sensitive element to an electrical signal, while a comparator circuit is present for comparing the measured signal generated by the signal transducer with a standard signal.

This known apparatus - see Figs 8-9 of EP-A-0.295.755 - has three striking elements mounted on the carrying frame, which are moved simultaneously in the direction of the egg by driving the carrying frame of the striking element in some manner or other. Because the egg to be tested will be contacted by all three striking elements simultaneously only in exceptional cases, the striking elements must be mounted in the carrying frame so as to be movable in axial direction.

In order to render this apparatus suitable for carrying out the method according to the invention described hereinabove, each striking element is capable of being driven individually in two opposite axial directions, i.e., to and away from the egg to be tested.

In this manner, each striking element can be placed in the reference position independently of the other striking elements, for testing the same egg and generating measured signals from that position.

To enable each striking element to be placed in its reference position, the driving means of a striking element preferably comprise means for determining the distance covered by a striking element in the direction of drive.

An elegant combination of driving and distance-measuring means is obtained by constructing it as a stepping motor and a friction wheel capable of being driven in two directions by the motor, this friction wheel bearing against a driving strip connected to the striking element.

The method according to the invention and an embodiment of an apparatus suitable for carrying out this method will now be further explained with reference to the accompanying drawings. In said drawings:

Fig. 1 is an elevation of the test apparatus, viewed in the direction of conveyance of the eggs to be tested;

Fig. 2 is a section taken on the line II-II of Fig. 3; and

Fig. 3 is a test station, viewed from above, equipped with a number of striking systems.

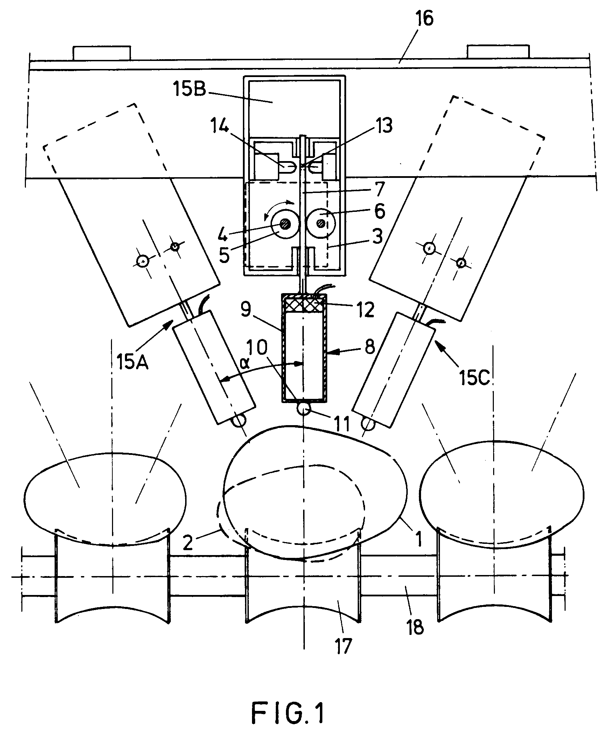

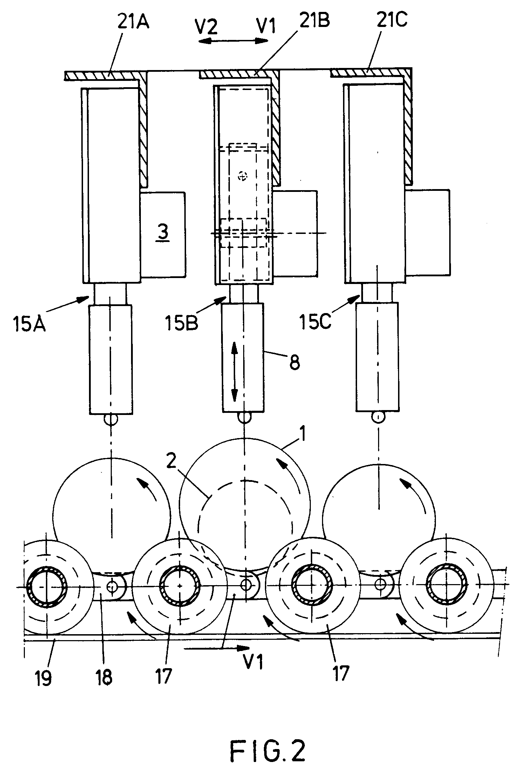

As appears from Figs 1 and 2, a testing apparatus according to the invention is arranged above a conveying apparatus 18 comprising a plurality of rollers 17, with each egg 1, 2 to be tested being rotatably supported by two rollers 17. The eggs 1, 2 have their longitudinal axes extending transversely to the direction of conveyance. Mounted on the frame 16 are a series of striking systems 15A, 15B, 15C directed at each egg, and capable of striking said egg on different diameters. Each striking system 15 comprises the actual striking element 8 with associated drive 3-7.

The striking element 8 is known per se from EP-A-0.295.755, mentioned above, and comprises a hollow tube 9 forming an air chamber, this tube being closed at the lower end by a vibration-sensitive element consisting of a membrane 10 having a ball 11 arranged in the centre thereof, which ball can be contacted with the egg shell if the striking element 8 is moved in downward direction. The hollow tube 9 is provided at the top thereof with a signal transducer 12, such as a microphone, capable of generating an electrical signal upon impact of the ball 11 against the egg 1, 2, which signal is a measure for the resilience of the egg shell. The amplitude and/or the frequency and/or the duration of this signal are compared with a known empirically determined signal profile of an intact egg shell in a comparator which is not further described here because it corresponds with the comparator according to EP-A-0.295.755.

The striking element 8 is driven by a stepping motor 3 (see Fig. 2). This stepping motor 3 is provided with a friction wheel 5, mounted on the output shaft 4 and bearing against a driving strip 7 at one end of which is mounted the striking element 8. Mounted opposite the friction wheel 5 is a freely rotatable counter wheel 6, arranged for supporting and guiding the driving strip 7. The driving strip 7 is provided at the other end thereof with an aperture 13.

The zero or starting position of the striking element 8 is determined with the aid of a lamp-photo cell combination 14 capable of detecting the aperture 13 in the driving strip 7 and generating an electrical signal for switching off the stepping motor 3. In this starting position, the striking element 8 is ready for carrying out a (next) test cycle.

At the beginning of the test cycle, the striking element 8 is moved from the starting position to the egg 1, 2 by means of the stepping motor 3. As soon as the striking element 8 contacts the egg shell, the signal transducer 12 generates a signal, with the distance between the starting position of the striking element 8 and the egg shell being determined by the number of steps of the stepping motor 3. Thus, the diameter of the egg 1, 2 at the location of the point of measurement is determined as well. By moving the striking element 8 backwards a certain number of motor steps and contacting the egg shell from that reference position, the resilience of the egg shell at a certain point is measured. Because the eggs on the conveyor 18 rotate about their longitudinal axes, the egg is contacted at different points of the circumference, the resilience of the egg shell being measured at that point. All these measurements together form a test cycle.

The frame 16 on which the striking systems 15A, 15B, 15C are mounted is provided with driving means (not shown) capable of moving the frame 16 at the same speed $V_1$ as the traveling speed of the conveyor 18. In this manner, the testing of eggs can take place during conveyance and the rollers 17 of the conveyor 18 need not be separately driven for rotation. The rollers 17 of the conveyor 18 are supported by a rail 19 and start rotating when the conveyor chain 18 is moved.

As shown in Fig. 2, the striking systems 15A, 15B, 15C can be arranged and mounted on the frame 16, behind each other, viewed in the direction of conveyance. In this manner, a plurality of eggs located behind each other on the conveyor 18 can be tested during one test cycle. Thus, the total time for one test cycle remains the same but the measuring capacity is increased considerably.

Fig. 1 shows a construction whereby an egg 1 can be struck simultaneously by the three striking systems 15A, 15B, 15C at different egg diameters. As a consequence, it may happen that the impact of, for instance, striking system 15A on the egg 1 is received not only by the vibration-sensitive member 10, 11 associated with that striking system 15A, but also by the vibration-sensitive member of the adjacent striking system 15B, so that the signal generated by the signal transducer 12 of that striking system 15B is no longer representative of the strength of the egg shell at the point of impact of striking system 15B. Moreover, the striking systems 15A, 15B, 15C arranged side by side occupy more space in lateral direction than the pitch distance between the eggs of a row, located side by side on the rollers 17, so that eggs located side by side must be measured by striking systems 15 that are arranged behind each other, as shown in Fig. 2.

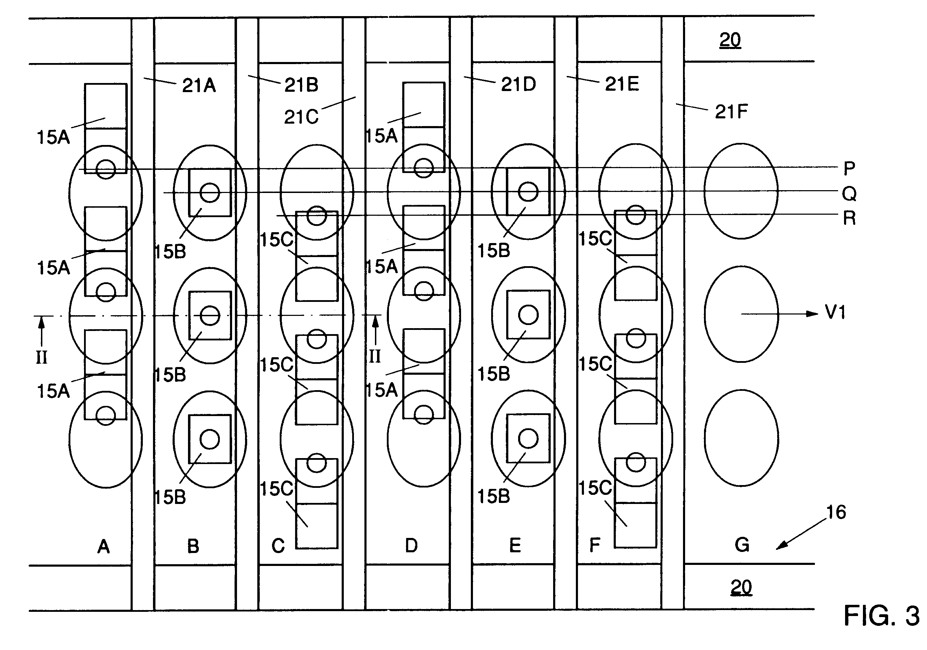

Fig. 3 is a top plan view of a detecting station that precludes the striking systems 15A, 15B, 15C from having any influence on each other. Fig. 3 shows seven rows A through G, located in succession on the conveyor 18. The carrying frame 16 mounted above the eggs consists of two longitudinal beams 20 and six transverse beams 21A-21F, arranged at the same mutual pitch distance as the distance between successive rows of eggs A-G. The transverse beams 21A and 21D are equipped with striking systems 15A (see Fig. 1), capable of striking the eggs at points located in the line P, i.e., left of the middle. The transverse beams 21B and 21E are equipped with striking systems 15B, capable of striking the eggs at the points located in the central diameter (line Q), while transverse beams 21C and 21F are equipped with striking systems 15C, capable of striking the eggs at the points located right of the middle (line R).

The operation of this apparatus is as follows. The conveying apparatus 18 and the carrying frame 16 both move at a speed $V_1$ in the direction indicated, while all striking systems 15A-15C are in operation and generate measuring signals which are compared with standard signals in the manner described above. The instantaneous position of "defective" eggs is stored in a memory in a manner that is known per se. These eggs are removed from the system at a later time, likewise in a manner that is known per se.

The eggs entering the detecting station are tested at row A by the striking system 15A. The eggs of row B are tested by the striking systems 15B at points located in the line Q. The eggs of row C are tested by the striking systems 15C at points located in the line R. These eggs of row C have previously been tested by the striking systems 15A of row A at points located in the line P, but are not tested at points located in the line Q until later, namely at row E. This means that the striking systems 15A mounted on the transverse beams 21A test alternate rows, i.e., these striking systems each time skip one row of eggs. Rows A-C-E and G have been tested by the striking systems 15A of transverse beam 21A at points located in line P, whereas rows B-D-F have been or will be tested by the striking systems 15A of transverse beam 21D at points likewise located in line P. The striking systems 15B mounted on the transverse beams 21B and 21E operate in identical manner and the same holds for the striking systems 15C mounted on the transverse beams 21C and 21F.

The cycle time $t_c$ of the system described above is therefore equal to the time it takes for the conveying apparatus 18 to cover the distance S, being twice the pitch distance between successive rows of eggs, i.e.,

$$t_c = \frac{s}{v_1},$$

wherein $V_1$ is the travelling speed of the conveyor 18. During the same interval, the carrying frame 16 must

- move along with the conveyor 18 at the speed $V_1$ for a period $t_m$ during which the measurements or tests are carried out; and
- travel back for a period $t_c-t_m$ at an increased speed $V_2$, to a new measuring position, shifted by two rows A-G relative to the previously occupied position.

If, for instance, $t_m = \frac{2}{3} t_c$, then a speed of $V_2 = 2 \times V_1$ is required for the rearward displacement of the carrying frame 16. If the measuring period $t_m$ is increased, the speed $V_2$ must be increased accordingly.

**Claims**

1. A method for detecting hair cracks or holes in egg shells, in which the egg shell to be tested is caused to vibrate locally by means of at least one striking element (8) equipped with a vibration-sensitive element (10, 11) and a signal transducer (12), the vibration being transmitted via the vibration-sensitive element (10, 11) to the signal transducer (12) and converted to an electrical signal whose amplitude and/or frequency and/or duration are a measure for the resilience of the egg shell, characterized in that the signal generated when the striking element (8) first strikes the egg (1, 2) is used for determining the egg diameter-dependent, variable distance between a fixed starting position of the striking element (8) and the egg shell, whereafter the striking element (8) is retracted over a certain fixed distance from the contact position to a reference position from which the striking element (8) is contacted again with the egg shell for generating a measuring signal.

2. An apparatus for carrying out the method according to claim 1, comprising a carrying frame (16) in which a series of striking systems (15A, 15B, 15C) provided with axially movable striking elements (8)are mounted, which striking elements have their centrelines directed to an egg shell (1, 2) to be tested and, at the end proximal to the egg shell, are provided with a vibration-sensitive element, each striking element (8) further comprising a signal transducer (12) for converting the vibration transmitted by the vibration-sensitive element (10, 11) to an electrical signal, while a comparator circuit is present for comparing the measured signal generated by the signal transducer (12) with a standard signal, characterized in that each striking element (8) is capable of being driven individually in two opposite axial directions, i.e., to and away from the egg (1, 2) to be tested.

3. An apparatus according to claim 2, characterized in that the driving means (3-7) of a striking element (8) also comprise means for determining the distance covered by a striking element (8) in the direction of drive.

4. An apparatus according to claims 2-3, characterized in that the driving and distance-measuring means consist of a stepping motor (3) and a friction wheel (5) capable of being driven in two directions by said motor (3), said friction wheel (5) bearing against a driving strip (7) connected with the striking element (8).

5. An apparatus according to claim 4, characterized in that each striking element (8) is equipped with means (14) capable of generating an electrical signal as soon as the driving strip (7) of the striking element (8) has reached its starting position.

6. An apparatus according to any one of claims 1-5, characterized in that driving means are present for driving the carrying frame (16) in the direction of conveyance of the eggs (1, 2) which are rotatably supported by an egg conveyor (18) arranged under the carrying frame (16), at the same speed ($V_1$) as the egg conveyor and at an increased speed ($V_2$) in opposite direction.

7. An apparatus according to claim 6, characterized in that a series of striking systems (15) are mounted on the carrying frame (16) behind each other, viewed in the direction of conveyance, at mutual distances corresponding with the pitch distance between the eggs (1, 2) disposed on the egg conveyor (18).

8. An apparatus according to claims 6-7, characterized in that the carrying frame (16) comprises a series of transverse beams (21A-21F), each transverse beam being equipped with a series of striking systems (15A; 15B; 15C) whose lines of action are substantially parallel to each other, while the lines of action of the striking systems (15B) mounted on a next transverse beam (21B) form an acute angle with the lines of action of the striking systems (15A) mounted on a preceding transverse beam (21A).

FIG.1

FIG.2

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 295 755 (TERPA POULTRY B.V.) 21 December  1988 * the whole document * | 1-8 | G01N33/08 A01K43/00 G01N29/04 |
| A | US-A-3 503 501 (SEABORN) 31 March 1970 * the whole document * | 1-8 | |

TECHNICAL FIELDS SEARCHED (Int. Cl.5 )

G01N
A01K

The present search report has been drawn up for all claims

| Place of search | Date of completion of the search | Examiner |
|---|---|---|
| THE HAGUE | 20 AUGUST 1993 | BOSMA R.A.P. |

EPO FORM 1503 03.82 (P0401)